# EUROPEAN PATENT APPLICATION

(11) **EP 2 926 686 A1**
(43) Date of publication of application: **07.10.2015**
(21) Application number: 14182991.1
(22) Date of filing: 01.09.2014
(51) Int. Cl.: A45D 40/24, G01N 31/22, A45D 40/18

(54) **Dispenser apparatus**

(30) Priority: 31.03.2014 US 201461972981 P; 01.07.2014 US 201414321398
(71) Applicant: Agent V, LLC, Wayzata, MN 55391 (US)
(72) Inventor: Cashman, Debra, Wayzata, MN Minnesota 55391 (US)
(74) Representative: Puschmann Borchert Bardehle Patentanwälte Partnerschaft mbB

(57) **Abstract**

An implement usable by a woman or gentleman to protect herself/himself against someone depositing a date rape drug in her/his beverage. The inventive implement includes a wet reservoir and a dry plenum in which products that are typically dispensed by the implement are stored.

## Description

The present invention deals broadly with structures, used by women or men, which are employed in applying cosmetics. More narrowly, however, the invention is directed to a dispenser by which one of various cosmetic products can be stored and applied by the user, and which reminds the user, mindful of the hazards of a social environment where alcoholic beverages may be served, to take action to protect herself from the potential consequences of her beverage being spiked without her knowledge. The invention deals with protecting women of all ages who go anywhere where alcoholic and nonalcoholic beverages are served. It allows and encourages a woman to be beautiful and confident that she is not being victimized.

Women go to parties, dance clubs, bars, campus parties, house parties, and travel and go on dates. A terrible problem of drink spiking, known by the slang word "roofie", has developed in society. Most women use lip gloss, lipstick or other applications and carry a dispenser of such a product with them in their purse. Many, however, are not sufficiently attentive to taking precautions against being victimized by a ruthless perpetrator using a drink-spiking agent.

A typical scenario is one in which one or more date rape drugs are administered to a victim either surreptitiously or where the victim has been disposed in a condition wherein she is unable to resist sexual assault. The encounter between the victim and the perpetrator may have been a casual first meeting between the two, a tryst between two people who have an established relationship or even with a total stranger.

In any case, the woman may be confronted by an event that requires her leaving her drink unattended for a short period of time for one of a variety of reasons. Typical are a desire to dance, respond to a page, or answer a phone call, or even merely turn her head. Also common is a desire to apply cosmetics. There are various treatment products and apparatus involved in applying cosmetics. These include a mascara-applying brush with a curved distal portion, a perfume applicator which includes an elongated applicator shaft, the end of which is dipped in a perfume reservoir, and various tools for the application of a product to one's lips. These include lipstick applicators, lip balm applicators, a shaft at the distal end of which a lip gloss pillow is attached, and a roller ball lip gloss applicator. Regardless of what tool is being used, however, there is the distraction of the primary objectives of safety and security.

As a result, her beverage is left unattended, perhaps even momentarily. She eventually returns to the bar or table, or redirects her attention to a man with whom she has been engaging in conversation. The man may, however, have ideas different than the woman. As he has been getting to know the woman, he recalls that one of his acquaintances had given him a tablet of Rohypnol, and he sees the woman's excusing herself as an opportunity to spike the beverage left unattended by her. When she returns she resumes drinking her beverage which has been spiked with any chemical agent having one or more of the consequences listed hereafter.

The effects of such drugs are felt relatively quickly. These can include, among others, sleepiness, dizziness, slurred speech and loss of memory of the events. In a relatively large number of such encounters, non-consensual sex does occur.

Rohypnol is only one of a number of agents which fall within the characterization of a date rape drug. There are other chemical agents which can also be so characterized. Flunitrazepam, also known as Narcozep, and others are available.

The present invention, as will be seen, is an improved dispenser for both cosmetics and means for testing whether a beverage has been spiked without the knowledge of the person whose beverage it is. More specific features and advantages obtained in view of those features will become apparent with reference to the summary of the invention.

The present invention is a dispenser for holding and dispensing two different products. The inventive implement includes an elongated body which defines, at a first end, a liquid reservoir and, at a second end, a dry plenum. Means are provided to interconnect the reservoir and the plenum. Typically, a yoke structure is used to interconnect the reservoir portion and the plenum portion. The yoke employs generally oppositely extending fittings which are matable to corresponding nipples defined at proximal ends of the liquid reservoir and the dry plenum. It is envisioned that, when the implement is in an assembled configuration, the fittings of the yoke and corresponding nipples are substantially coaxial.

It is envisioned that the plenum and reservoir, together, form an elongated structure. The plenum portion of the structure is sized so as to enable it to receive drink-spike detector test strips. Such strips are of a type that are chemically impregnated, and, when dipped in a beverage, for example, project a conclusion as to whether the drink possesses any chemical agent known as a "date rape" drug.

The present invention is thus an improved structure for assisting a woman in knowing whether a beverage she is consuming has had any drugs deposited therein. More specific features and advantages obtained in view of those features will become apparent with regard to the detailed description of the Invention and the appended claims.
- FIG. 1: is a perspective view of the invention;
- FIG. 2: is a side view section thereof taken along line 2-2 in FIG. 1;
- FIG. 3: is a view similar to that of FIG. 2 with the plenum portion containing test strips disconnected; and
- FIG. 4: is a view thereof with the dispenser angled downward to dispense test strips, and one test strip shown removed in close proximity thereto.

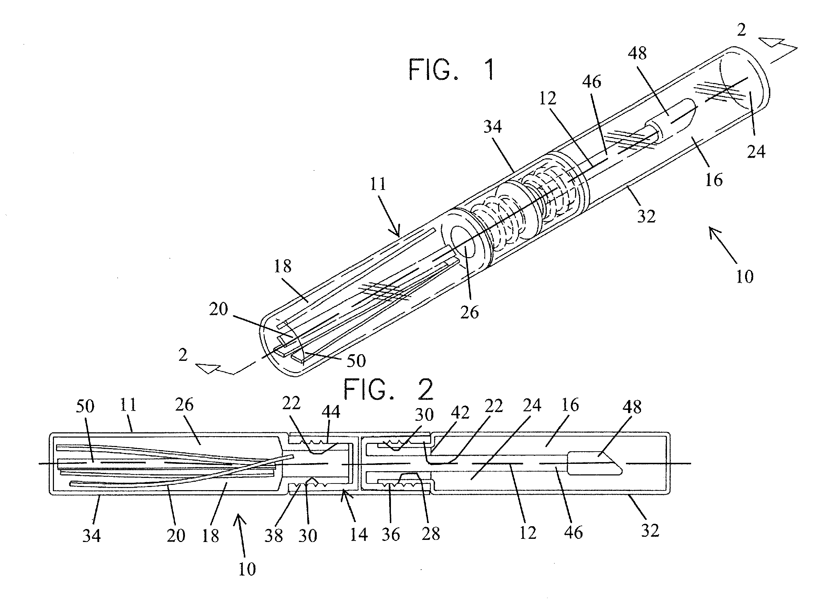

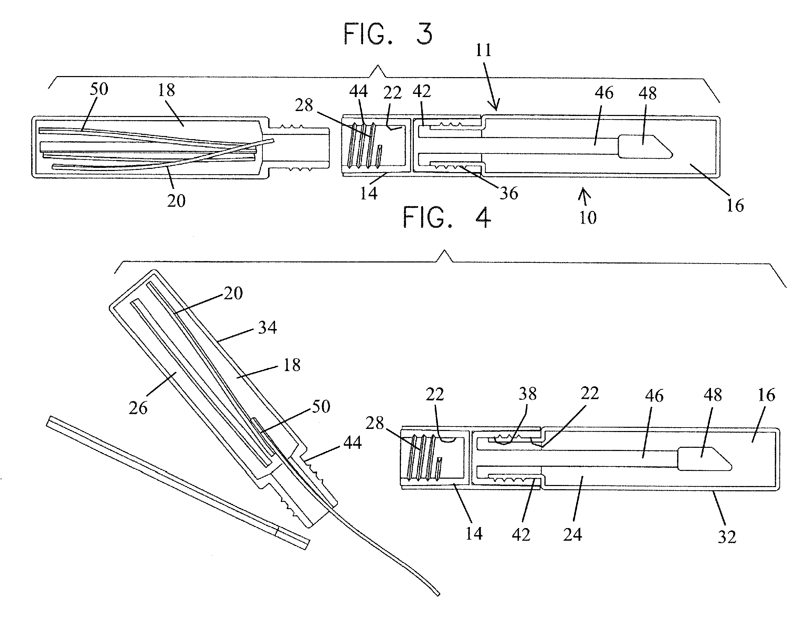

Referring now to the drawing figures wherein like reference numerals denote like elements throughout the several views, FIGS. 1-4 show an implement 10 in accordance with the present invention. The implement 10 is a dispenser 11 for cosmetics. Unlike dispensers known in the prior art, however, it includes separate spaces, typically aligned along an axis, for storing defined materials.

As illustrated in the figures, the overall implement 10 is elongated with respect to an axis 12 which extends along the elongated dimension. As best seen in FIG. 1, the overall implement 10 can be generally circularly cylindrical when assembled. When in such a configuration, the diameter of the implement 10 is substantially less than the elongated dimension. This enables facile storage in a purse or other compact container (not shown).

Referring to FIGS. 2-4, it will be seen that structurally the implement 10 includes a yoke 14 generally central with respect to the axial dimension. The yoke 14 defines a portion of a liquid reservoir section 16 of the implement 10 and a portion of a plenum section 18 for typically holding dry materials (as at 20). It is intended that an inner wall 22 defining each of these sections 16, 18 of the reservoir 24 and plenum 26 be internally threaded (as at 28, 30) so as to enable receipt of major walls 32, 34 defining the reservoir 24 and plenum 26 respectively.

The reservoir 24 and plenum 26 include portions which are externally threaded (as at 36, 38) so as to enable them to be mated with their respective sections of the containment spaces 24, 26. Neck portions 42, 44 define access ports through which a material, such as lip gloss, can be dispensed. With the reservoir 24 substantially full, the neck 42 of the reservoir component is screwed tightly into the small axial neck 42 defined by the yoke 14.

In the case of the reservoir 24, the neck portion 42 thereof includes, extending generally along the axis, a shaft 46 which, when the reservoir 24 is attached to the yoke 14, has a distal end 48 which extends substantially the full axial length of the reservoir 24. Such a construction will facilitate access to any fluid at the distal end 48 of the reservoir.

Section 18 of the dispenser 11 will, through its neck portion 44, enable insertion and removal of a typically constructed chemical test strip 50. In fact, multiple strips 50 can be allowed to be received within the plenum 26. Access to the strips 50 is afforded through the neck 44 of the plenum component 26 when the neck thereof has been unthreaded from the internal threading in the yoke 14.

With regard to use of the dispenser 11, a user of the invention, if she, for example, excuses herself to visit the ladies' room in order to apply a cosmetic to freshen up, she can feel secure when she returns by using a test strip 50 removed from the dry plenum 26 and inserting all or part of the strip 50 into a drink (not shown) which she has left on the bar during her absence. While in the ladies' room, she has obtained access to the liquid reservoir 16 of the implement 10 by unthreading threads 28. Distal end 48 of shaft 46 became exposed and permitted application of the cosmetic. Now, having performed the beautification function, the testing of her beverage can be performed. Access to the dry plenum section 18 is performed by unthreading neck portion 44 from the threads 28 in the yoke 14. Testing using the strip can be performed either surreptitiously or demonstratively. In either case, having performed the testing can give the woman a certainty as to the status of her beverage.

It will be understood that this disclosure, in many respects, is only illustrative. Changes may be made in details, particularly in matters of shape, size, material, and arrangement of parts without exceeding the scope of the invention.

## Claims

1. Dispenser apparatus for storing and dispensing various products frequently utilized by an individual, comprising:
a) an elongate implement body defining, as a first end thereof, a first space for a first product and, as a second end thereof, a second space for a second product; and
b) means for interconnecting said first space and said second space.

2. Apparatus in accordance with Claim 1 wherein said means for interconnecting comprises a yoke having generally oppositely extending fittings, each fitting matable to a corresponding nipple defined at a proximal end of one of said first space and said second space.

3. Apparatus in accordance with Claim 2 wherein said fittings are internally threaded and wherein said nipples are externally threaded.

4. Apparatus in accordance with Claim 2 wherein said fittings and said nipples are, when the apparatus is in an assembled configuration, substantially coaxial.

5. Apparatus in accordance with Claim 1 wherein the first product is a liquid product.

6. Apparatus in accordance with Claim 5 wherein the liquid product is a cosmetic product.

7. Apparatus in accordance with Claim 1 wherein the second product is a drink-spike detector means.

8. Apparatus in accordance with Claim 1 wherein the second space is sized to received drink-spike detector means.

9. Apparatus in accordance with Claim 8 wherein said drink-spike detector means comprises a plurality of chemically impregnated test strips.

10. Apparatus in accordance with Claim 1 for holding first and second different products, and for providing access to either of the products by a user, comprising:
a) a first cylindrical chamber defining the first space for holding the first product, and having an opening at a proximal end thereof, said first opening having a first attachment structure;
b) a second cylindrical chamber defining the second space for holding the second product, and having an opening at a proximal end thereof, said second opening having a second attachment structure;
c) an interconnecting element having, at a first end, a first mating structure for mating with the first attachment structure, and for detachably retaining the first attachment structure, and, at a second end, a second mating structure for mating with the second attachment structure and for detachably retaining the second attachment structure.

11. Apparatus in accordance with Claim 10, wherein at least one of the first mating structure and the first attachment structure, and the second mating structure and the second attachment structure, telescope with respect to each other.

12. Apparatus in accordance with Claim 1 for storing and dispensing drink-spike test strips, comprising:
a) a dry plenum as the first space sized to receive a plurality of conventional drink-spike test strips, said dry plenum having an egress port at a proximal end thereof;
b) a reservoir as the second space for receiving a cosmetic product, said reservoir having an egress port at a proximal end thereof, and wherein said plenum and reservoir egress ports are substantially axially aligned and spaced relative to one another; and
c) means for interconnecting said proximal ends of said plenum and said reservoir.

13. Apparatus in accordance with Claim 12 wherein said interconnecting means comprises a yoke.
